(19) Europäisches Patentamt / European Patent Office / Office européen des brevets

(11) **EP 4 008 244 A1**

(12) **EUROPEAN PATENT APPLICATION**

(43) Date of publication:
**08.06.2022 Bulletin 2022/23**

(21) Application number: **20210971.6**

(22) Date of filing: **01.12.2020**

(51) International Patent Classification (IPC):
**A61B 5/01** (2006.01)       **A61B 5/11** (2006.01)
**A61B 5/0537** (2021.01)     **A61B 5/00** (2006.01)

(52) Cooperative Patent Classification (CPC):
**A61B 5/01; A61B 5/0537; A61B 5/1101;**
**A61B 5/1118; A61B 5/1128; A61B 5/4266;**
**A61B 5/4842; A61B 5/7275; A61B 5/7278;**
A61B 5/1123; A61B 5/4839; A61B 5/7221;
A61B 5/7257; A61B 2560/0238; A61B 2560/0252

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **HUIJBREGTS, Laurentia Johanna**
  **5656 AE Eindhoven (NL)**
• **JOHNSON, Mark Thomas**
  **5656 AE Eindhoven (NL)**
• **VAN LIESHOUT, Ron Martinus Laurentius**
  **5656 AE Eindhoven (NL)**
• **GERHARDT, Lutz Christian**
  **5656 AE Eindhoven (NL)**
• **PELSSERS, Eduard Gerard Marie**
  **5656 AE Eindhoven (NL)**

(74) Representative: **Philips Intellectual Property &**
**Standards**
**High Tech Campus 52**
**5656 AG Eindhoven (NL)**

(54) **FEVER PROGRESSION MONITORING**

(57)     A computer-implemented method (100) is disclosed for monitoring progression of a fever in a subject (1). The computer-implemented method comprises receiving (103, 109) input data indicative of shivering episodes (52) and sweating episodes (54) of said subject as indicators of progression of a fever in said subject over a time interval; recording (105, 111) the respective points in time at which said shivering episodes and sweating episodes occur; and generating (115, 117) an output indicative of progression of fever in said subject based on an alternating pattern of said shivering episodes and sweating episodes from the recorded respective points in time of the respective shivering and/or episodes. Also disclosed is a computer program product for implementing such a method and a monitoring system (10) arranged to implement such a method.

FIG. 1

EP 4 008 244 A1

**Description**

FIELD OF THE INVENTION

The present invention relates to a computer-implemented method for monitoring progression of a fever in a subject such as a patient, the computer-implemented method comprising, with a processor arrangement, receiving input data for monitoring said fever progression.

The present invention further relates to a computer program product for implementing such a method on a monitoring system, and a monitoring system arranged to implement such a method.

BACKGROUND OF THE INVENTION

Fever is defined in a patient as the patient having a core body temperature (CBT) above the normal range due to an increase in the body's temperature set point. A fever can be caused by many medical conditions ranging from non-serious to life threatening. This includes viral, bacterial and parasitic infections such as the common cold, urinary tract infections, meningitis, malaria, and appendicitis among others. Non-infectious causes include vasculitis, deep vein thrombosis, side effects of medication, and cancer among others. Fever is considered advantageous to the host in surviving bacterial infections and is regulated by the thermoregulation methods of the body, where in particular sweating and shivering play a major role.

WO 2017/172837 A1 discloses systems, methods and devices for utilizing heat transfer parameters or energy expenditure of devices providing controlled hypothermia, normothermia or hyperthermia to detect changes, or the absence of changes, to a patient's endogenous set-point temperature. When the body's core temperature deviates below an interthreshold zone, a series of coordinated responses occur that include surface vasoconstriction, shivering, and metabolic thermogenesis, among others. When the body's core temperature deviates above the interthreshold zone, a series of coordinated responses occur that include surface vasodilatation and sweating, among others.

Depending on the nature of the medical condition, the CBT of the patient may exhibit different time-dependent behavior. For example, fever linked to infection is caused by the increased set point due to cytokines released in the blood stream by circulating leukocytes, but when the source of infection, or in other words, when the activation of the host immune system is decreased, the concentration of cytokines in the blood stream decreases leading to a reduced inhibition in the hypothalamus, which leads to a lower set point and hence sweating to reach to lower set point, and as a consequence to a CBT that exhibits cyclic behavior of fever episodes over time. The CBT monitored over a prolonged period of time is also known as a fever curve, which can provide diagnostically relevant information to a medical professional such as a clinician or a nurse.

In high-acuity settings in the hospital, in particular in the Intensive Care Unit (ICU), CBT may be measured continuously. This is done via an invasive measurement. In lower-acuity settings, in particular on the general ward, it is desired to refrain from invasive techniques, e.g. because of infection risk. As there is no established reliable continuous non-invasive technique to measure CBT, CBT is measured during spot checks that commonly only take place one to three times per day. As a result, no fever curve can be reconstructed. Hence, there exists a need for non-invasive (semi-)continuous monitoring of a patient's CBT to allow the construction of such a fever curve for the patient.

SUMMARY OF THE INVENTION

The present invention seeks to provide a computer-implemented method for monitoring progression of a fever in a subject in a non-invasive manner such that a (surrogate) fever curve may be constructed for the subject.

The present invention further seeks to provide a computer program product for implementing such a method on a monitoring system, and a monitoring system for monitoring a subject, the monitoring system being arranged to implement such a method.

According to an aspect, there is provided a computer-implemented method for monitoring progression of a fever in a subject, the computer-implemented method comprising, with a processor arrangement, receiving input data indicative of shivering episodes and sweating episodes of said subject as indicators of progression of a fever in said subject over a time interval; recording the respective points in time at which said shivering episodes and sweating episodes occur; and generating an output indicative of progression of fever in said subject based on an alternating pattern of said shivering episodes and sweating episodes from the recorded respective points in time of the respective shivering and/or sweating episodes.

Embodiments of the present invention are based on the insight that detection of shivering and sweating episodes of a subject such as a patient or any other being at different points in time during a monitoring period, i.e. the time interval, can be interpreted as indicators of a change in CBT of the subject, i.e. the progression or change of a status of a fever in the subject, without having to record the actual CBT of the subject. In some embodiments, by recording the points in time at which these shivering and sweating episodes occur, a fever curve can be constructed for the subject, such that an output based on such a fever curve may be used for evaluation by a medical professional to obtain diagnostically relevant information about the progression of fever in the subject, which for instance may assist the medical professional in recognizing the type of medical condition of the subject, in defining a medication regime for the subject or in assessing the effectiveness of an applied medication regime, e.g. by monitoring changes in the fever curve after administration of the medication. Such a fever curve may be considered a surrogate fever curve because the curve is not based on actual CBT measurements but rather is based on inferences of changes in the subject's CBT from the aforementioned detection of shivering and sweating episodes.

In an embodiment, the computer-implemented method further comprises, with said processor arrangement, determining a periodicity of said alternating pattern of said detected shivering and sweating episodes based on their respective occurrence at different points in time during said time interval; and identifying a type of fever from the determined periodicity of said alternating pattern. By identifying the type of fever, e.g. based on the time lapse between subsequent shivering episodes, subsequent sweating episodes and/or a shivering episode and a subsequent sweating episode or vice versa, additional diagnostically relevant information can be extracted from the fever curve and presented to the medical professional, e.g. to aid the medical professional in the effective treatment of the subject's medical condition.

Such points in time may be recorded by a user manually generating the input data, e.g. an acknowledgement signal or the like, during the occurrence of a shivering or sweating episode of the subject. However, in a particularly advantageous embodiment, receiving said input data comprises receiving subject monitoring data from a sensor arrangement for monitoring shivering and sweating of said subject as indicators of progression of a fever in said subject over said time interval; and processing said received subject monitoring data to detect shivering and sweating episodes and record the respective points in time at which said detected shivering episodes and patent sweating episodes occur in the subject monitoring data such that no manual inputs to monitor the subject are required.

The computer-implemented method may further comprise, with said processor arrangement, determining at least one of a duration and a severity of each shivering episode and sweating episode; and calculating an indication of an intensity of the fever from the determined at least one of a duration and a severity of each shivering episode and sweating episode. This may provide valuable insights in the subject's ability to fight the fever, e.g. when caused by an infection, as expressed by the trends in the severity and/or the duration of such episodes overtime.

In a further refinement, the subject monitoring data may comprise information pertaining to a degree of thermal insulation of the subject, and the computer-implemented method may further comprise, with said processor arrangement, determining at least one of a duration and a severity of each shivering episode and sweating episode as a function of said information pertaining to a degree of thermal insulation of the subject. For example, whether the subject is covered by one or more blankets and/or wears a certain amount of clothing may have an impact on the severity and duration such that detection of these external influences can be used to more accurately assess the true nature of the shivering or sweating episode.

In a particular embodiment, the computer-implemented method further comprises, with said processor arrangement, determining a shivering frequency for each shivering episode; and determining if said shivering episode is an indicator of a progression of fever in said subject based on the determined shivering frequency. This is based on the insight that the shivering frequency caused by the onset of fever differs from the frequency of other involuntary shivering-like body motions, e.g. tremors or the like, thereby further ensuring that the shivering episodes included in the fever curve are genuinely associated a change in a subject's CBT caused by the onset of fever.

Preferably, a sweating episode is identified based on a sweat rate of said subject as this is a particularly accurate way of determining such a sweating episode. For example, the computer-implemented method may further comprise, with said processor arrangement, determining a sweat base rate for said subject from the received subject monitoring data during a shivering episode; and detecting a sweating episode by detecting an actual sweat rate of said subject in the received subject monitoring data that exceeds the sweat base rate by a defined amount. This is particularly advantageous as it provides a subject-specific baseline for the sweat rate based on the insight that during a shivering episode the subject's sweat rate is typically minimal, such that any sweat rate that is significant to the subject can be detected by comparing an actual sweat rate against this base rate, thereby making the detection method robust against varying sweat rates between subjects.

Alternatively or additionally, the computer-implemented method may comprise, with said processor arrangement, comparing an actual sweat rate of the subject against a defined sweat rate threshold; and detecting a sweating episode upon the actual sweat rate exceeding the defined sweat rate threshold to ensure that the detected sweat rate is sufficiently high such that its detection can be associated with a genuine sweating episode indicative of a lowering of the subject's CBT, i.e. the subsidence of a fever in the subject.

In a further embodiment, the computer-implemented method further comprises, with said processor arrangement, determining a signal strength of a signal pertaining to shivering episodes over the time interval; determining a total amount of sweat produced by said subject during the sweating episodes over the time interval; determining the subject's sweat rate over the time interval from the determined total amount of sweat produced by said subject; calculating a ratio of said determined signal strength and determined sweat rate; and including said calculated ratio in said output. This ratio may be used to reconstruct the fever curve to enable prediction of the severity of the fever and medication response efficacy for example.

The subject monitoring data may further comprise core body temperature data pertaining to the subject's core body temperature measured over said time interval with a core body temperature sensor of said sensor arrangement, wherein the computer-implemented method may further comprise, with said processor arrangement, including the subject's core body temperature data in said output. This for example allows for the determination of a lag between the occurrence of a shivering or sweating episode and the associated change in the subject's CBT as monitored by the core body temperature sensor, from which the subject's thermoregulatory ability can be derived. Moreover, the CBT measurement may assist in distinguishing between a shivering episode caused by the onset of fever, where this causes the subject's CBT to increase and a shivering episode caused by the subject being cold, where the shivering episode will not lead to an increase in the subject's temperature set point. This for instance may be used to verify whether the shivering episode can be reliably associated with the start of a fever episode, thereby further improving the accuracy of the subject monitoring. Moreover, inclusion of the CBT information, e.g. over a limited amount of time such as a time period covering a few fever cycles can be used for calibrating or training the construction of the fever curve by the processor arrangement. For example, the CBT data may be used as a ground truth to train artificial intelligence or machine learning algorithms. Such calibration or training thus can lead to the generation of a (surrogate) fever curve in which actual CBT can be accurately estimated. Such CBT information for example may be collected during spot checks or when the subject is being continuously monitored in an intensive care setting such as during an invasive procedure.

In another embodiment, the computer-implemented method further comprises, with said processor arrangement, receiving auxiliary information during said time interval through said communication interface arrangement, said auxiliary information comprising at least one of an ambient temperature to which the subject is exposed and a measurement of an activity level of the subject; determining whether a shivering episode or a sweating episode is a reliable indicator of progression of a fever in said subject based on the actual auxiliary information at the point in time of said shivering episode or sweating episode; and including the shivering episode or a sweating episode in generating said output only when said shivering episode or a sweating episode is determined to be a reliable indicator of progression of a fever in said subject. This facilitates the exclusion of shivering or sweating episodes that are triggered by factors other than fever from the fever curve, thereby increasing the accuracy of the fever curve.

According to another aspect, there is provided a computer program product comprising a computer readable storage medium having computer readable program instructions embodied therewith for, when executed on a processor arrangement of a monitoring system of a subject, cause the processor arrangement to implement the method of any of the herein described embodiments. Such a computer program product may be used to configure a monitoring system such that it can implement the method according to embodiments of the present invention.

According to yet another aspect, there is provided a monitoring system for monitoring a subject, the monitoring system comprising a processor arrangement arranged to receive input data indicative of shivering episodes and sweating episodes of said subject as indicators of progression of a fever in said subject over a time interval, wherein the monitoring system further comprises the computer program product of any of the herein described embodiments and the processor arrangement is arranged to execute said computer readable program instructions. Such a monitoring system therefore is capable of generating a fever curve of a subject without having to collect CBT information from the shivering and sweating episodes of the subject as captured in the subject monitoring data provided by the sensor arrangement, in which the monitoring system may provide the output based on the fever curve, e.g. an output including the fever curve and/or diagnostically relevant information derived from said fever curve, to a user interface such that the output may be assessed on the user interface, e.g. by a medical professional, or may store the output in a data storage architecture such as an electronic patient record for retrieval at any suitable point in time, e.g. by a medical professional.

The monitoring system may further comprise a communication interface arrangement arranged to receive said input data in the form of subject monitoring data from a sensor arrangement for monitoring shivering and sweating as indicators of progression of a fever in said subject and to forward said subject monitoring data to said processor arrangement, the monitoring system optionally comprising the sensor arrangement such that the subject may be monitored without requiring manual intervention, thereby providing a complete solution for subject fever progression monitoring based on the monitoring of the subject's shivering and sweating episodes as explained above. The sensor arrangement may comprise at least one of a camera, a sensor pad for integration in a bed, a wearable sensor including a sweat sensor and a motion sensor for detecting shivering, and preferably the sensor arrangement comprises said wearable sensor.

## BRIEF DESCRIPTION OF THE DRAWINGS

Embodiments of the invention are described in more detail and by way of non-limiting examples with reference to the accompanying drawings, wherein:

FIG. 1 schematically depicts a monitoring system according to an embodiment;

FIG. 2 schematically depicts an example fever curve (core body temperature on y-axis, time on x-axis);

FIG. 3 schematically depicts an aspect of the monitoring system of FIG. 1 in more detail;

FIG. 4 shows a flowchart of a fever progression monitoring method according to an embodiment;

FIG. 5 depicts a graph explaining the construction of a surrogate fever curve according to an example embodiment;

FIG. 6 depicts a graph explaining the construction of a surrogate fever curve according to another example embodiment;

FIG. 7 depicts a graph explaining the construction of a surrogate fever curve according to yet another example embodiment;

FIG. 8 shows a flowchart of an aspect of a fever progression monitoring method according to an embodiment; and

FIG. 9 is a graph (reconstructed core body temperature index on y-axis, time on x-axis) depicting several fever index curves.

## DETAILED DESCRIPTION OF THE EMBODIMENTS

It should be understood that the Figures are merely schematic and are not drawn to scale. It should also be understood that the same reference numerals are used throughout the Figures to indicate the same or similar parts.

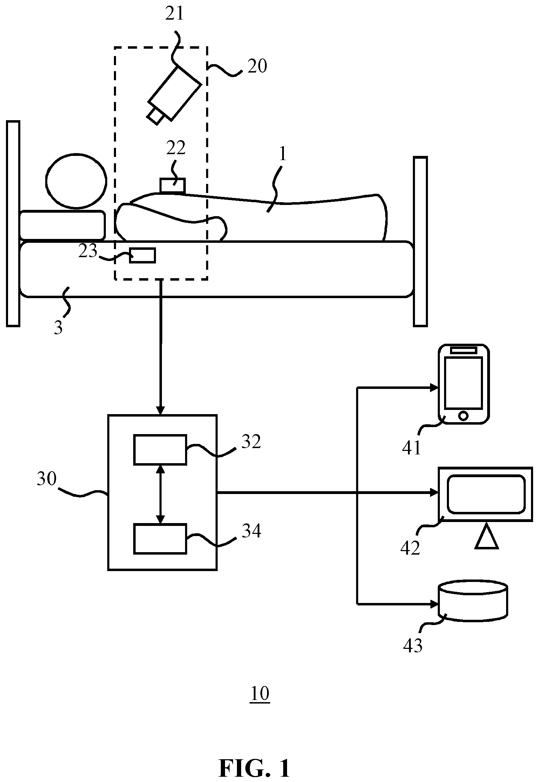

FIG. 1 schematically depicts an arrangement for monitoring the progression of a fever in a subject 1 with a monitoring system 10. The monitoring system 10 typically comprises a data processing architecture 30, e.g. a computer system, server system or the like, including a communication interface arrangement 32 in communication with a processor arrangement 34. The communication interface arrangement 32 may comprise any suitable number of data communication interfaces such as one or more P2P communication interfaces such as a Bluetooth interface, one or more wireless communication interfaces such as a WiFi interface, one or more wired communication interfaces such as an Ethernet interface, e.g. for connecting the data processing architecture 30 to a local area network, the Internet or the like, and so on. The processor arrangement 34 may comprise one or more processing elements such as one or more processors, processor cores and the like, arranged to process subject monitoring data provided by the sensor arrangement 20.

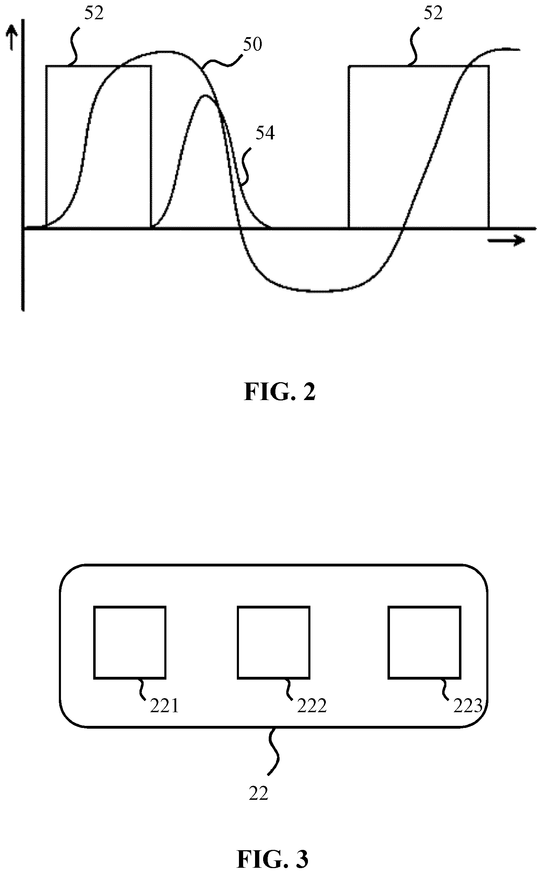

The sensor arrangement 20, which may form part of the monitoring system 10, is typically arranged to provide subject monitoring data that allows the processor arrangement 34 to detect shivering episodes 52 and sweating episodes 54 of the subject 1 being monitored in order to allow the processor arrangement 34 to construct a (surrogate) fever curve 50 for the subject 1 as schematically depicted in FIG. 2. This will be explained in further detail below. The subject monitoring data is typically provided to the data processing architecture 30 over more or more data communication links between the sensor arrangement 20 and the communication interface arrangement 32, which communication interface 32 forward the received subject monitoring data to the processor arrangement 34. In order to provide subject monitoring data from which the processor arrangement 34 can derive shivering and sweating episodes of the subject 1, the sensor arrangement 20 may comprise one or more sensors for this purpose.

For example, the sensor arrangement 20 may include a camera 21 aimed at the subject 1 to detect subject shivering, e.g. through the detection of subject motion in the images provided by the camera 21, and sweating, e.g. through changes in light reflection caused by the formation of sweat droplets on an exposed part of the subject's skin such as the subject's forehead as detected in the images provided by the camera 21. The sensor arrangement 20 may comprise a wearable sensor 22 that may be attached to the subject's skin for providing the required subject monitoring data. An example embodiment of such a wearable sensor 22 is shown in FIG. 3, which depicts a wearable sensor 22 comprising a motion sensor 221 such as an EMG or ECG sensor, an accelerometer or the like through which subject motion, e.g. shivering, can be detected, a sweat sensor 222 through which sweat production in a region of the subject's skin to which the wearable sensor 22 is attached can be detected, and optionally at least one additional sensor 223, for providing additional sensor data as will be explained in further detail below.

The sweat sensor 222 may be arranged to measure a sweat flow rate or a sweat volume after a defined collection time, or alternatively may be arranged to measure a sweat parameter indirectly, e.g. by measuring skin conduction. The wearable sensor 22 when including both the motion sensor 221 and the sweat sensor 222 should be placed on a part of the subject's body where both shivering and sweating can be reliably measured, such as on the subject's thorax or neck. The sensor arrangement 20 may comprise a sensor 23 for integration in the bedding, e.g. the mattress 3 on which the subject 1 is lying, for detection of subject motion whilst the subject 1 is in the bed, from which shivering may be detected. Any suitable combination of such sensors 21, 22, 23 may be deployed. In a preferred embodiment, the sensor arrangement 20 at least comprises the wearable sensor 22.

The data processing architecture 30 may further be communicatively coupled to one or more user interfaces 41, 42, on which the processing result of the subject monitoring data processing by the processor arrangement 34, e.g. the fever curve 50, may be displayed. To this end, the processor arrangement 34 typically generates an output based on this processing result, e.g. including this processing result or information derived from this processing result, which output may be forwarded to a particular user interface 41, 42 via the communication interface arrangement 32. For example, the user interface 41 may be a mobile communication device comprising an app or the like for visualizing the processing result of the processor arrangement 34, which may be used by a medical professional such as a nurse or doctor to evaluate the fever curve of the subject 1. The user interface 42 may be a user terminal such as a personal computer or the like through which the medical professional may receive the processing result from the processor arrangement 34. Alternatively or additionally, the processor arrangement may be arranged to store the processing result in a data storage arrangement 43, e.g. a network-connected database or the like storing the subject's monitoring data, e.g. an electronic patient record or the like, such that the processing result may be accessed at any suitable point in time, e.g. using the user interface 41 or 42. Other suitable architectures will be apparent to the skilled person.

In an alternative embodiment (not shown), the sensor arrangement 20 may be omitted and the processor arrangement 34 may instead by arranged to receive manually generated input data, e.g. by the subject 1 or by a medical caregiver such as a nurse or doctor, using an input device such as a remote control, an electric communication device such as a smart phone, or the like, such that the person operating such an input device can signal the occurrence of a shivering episode 52 or a sweating episode 54 of the subject 1 with the input device, such that the processor arrangement only has to record the point in time at which such an input has been received to construct the fever curve 50.

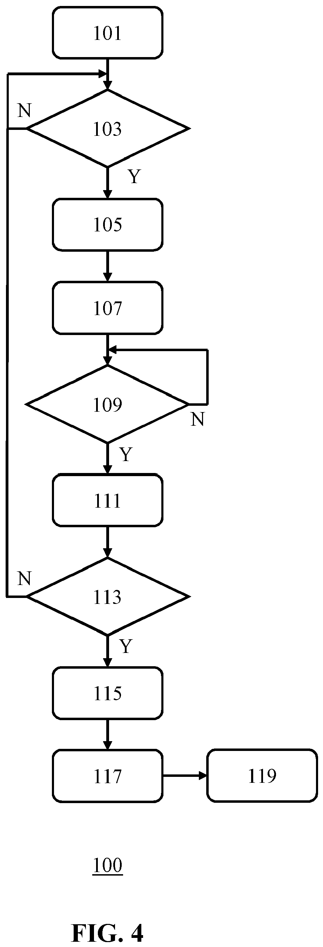

The monitoring system 10 is arranged to implement one or more of the embodiments of the method 100 of the present invention, a flowchart of which is shown in FIG. 4. The method 100 starts in operation 101, for example in which the sensor arrangement 20, if present, is activated and provides the previously described subject monitoring data to the processor arrangement 34 through the communication interface arrangement 32. In operation 103, the processor arrangement 34 processes the subject monitoring data received from the sensor arrangement 20 to determine if a shivering episode 52 of the subject 1 can be detected in the subject monitoring data. This processing may take place continuously or periodically, i.e. the subject monitoring data may be processed on a continuous basis or may be sampled periodically, e.g. once every 1- 5 minutes, for processing by the processor arrangement 34. If no shivering is detected, the processor arrangement 34 continues to look for a shivering episode 52 of the subject 1 in the received subject monitoring data. However, if the processor arrangement 34 detects the occurrence of such a shivering episode 52, the method proceeds to operation 105 in which the processor arrangement records the point in time at which the shivering episode 52 was detected, e.g. as derived from the subject monitoring data provided by the sensor arrangement 20 or as derived from manually generated input data flagging a shivering episode 52. This point in time may be expressed in any suitable format, e.g. as the amount of time lapsed after commencement of the subject monitoring, as the actual time of day or the like.

The method 100 may proceed to operation 107, in which the processor arrangement 34 determines a sweat base rate for the subject 1 during the shivering episode 52. This is based on the insight that during such a shivering episode 52, the CBT, i.e. the temperature set point, of the subject 1 is increasing, which typically equates to minimal associated sweating. Therefore, determining a sweat rate during such a shivering episode 52 provides a reliable baseline for determining a subsequent sweating episode 54 of the subject 1, as an actual sweating episode 54 of the subject 1 would lead to a significantly higher sweat rate than the baseline sweat rate. The determination of such a baseline sweat rate has the advantage that a subject-specific sweat rate baseline is defined, such that there is no need to account for different sweat rates in different subjects in order to reliably detect a sweating episode of the subject 1. However, in alternative embodiments the detection of such a sweat base rate may be omitted, in which case a sweating episode 54 in a subject 1 may be detected using a defined sweat rate threshold, which may be generic, gender-specific, specific to a location of the wearable sensor 22 on the subject's body, and so on.

In operation 109, the processor arrangement 34 processes the subject monitoring data received from the sensor arrangement 20 to determine if a sweating episode 54 of the subject 1 can be detected in the subject monitoring data. This processing may take place continuously or periodically, i.e. the subject monitoring data may be processed on a continuous basis or may be sampled periodically, e.g. once every 1 to 5 minutes, for processing by the processor arrangement 34. For example, the processor arrangement 34 may determine an actual sweat rate of the subject 1 and may compare this actual sweat rate against the sweat base rate determined in operation 107 and determine that a sweating episode 54 is occurring if the actual sweat rate exceeds the sweat base rate by a defined amount, e.g. a defined factor such as a factor 4-6.

Alternatively or additionally, the processor arrangement 34 may compare the actual sweat rate against a defined sweat rate threshold and determine that a sweating episode 54 is occurring if the actual sweat rate exceeds the defined threshold to ensure that the sweat rate is sufficiently high, e.g. above 0.7 nL/min for a sweat gland, to decide that an sweating episode 54 is occurring with sufficiently high confidence. Such a sweat rate may be determined using the wearable sensor 22, e.g. by determining an actual sweat rate or a parameter associated with changes in sweat rate such as skin conductance, or with the camera 21, e.g. by determining a change in reflected light from the camera images and deriving the sweat rate from this change.

If no sweating episode 54 is detected, the processor arrangement 34 continues to look for a sweating episode 54 of the subject 1 in the received subject monitoring data in operation 109. However, if the processor arrangement 34 detects the occurrence of such a sweating episode, e.g. in the subject monitoring data provided by the sensor arrangement 20 or as derived from manually generated input data flagging a sweating episode 54, the method proceeds to operation 111 in which the processor arrangement 34 records the point in time at which the sweating episode 54 was detected. This point in time may be expressed in any suitable format, e.g. as the amount of time lapsed after commencement of the monitoring of the subject 1, as the amount of time lapsed after the previously detected shivering episode 52, as the actual time of day, or the like.

As expressed in operation 113, the recording of the points in time at which shivering episodes 52 and sweating episodes 54 may continue, in which case the method 100 returns to operation 103 in which the processor arrangement 34 processes the subject monitoring data to detect the next shivering episode 52 or alternatively awaits the manual flagging of such an episode as previously explained. The method 100 may instead proceed to operation 115 in which the processor arrangement 34 constructs the fever curve 50 for the subject 1 from the recorded points in time at which the respective shivering episodes 52 and sweating episodes 54 of the subject 1 occurred. It is noted for the avoidance of doubt that the construction of the fever curve 50 may be performed after monitoring the subject 1 over a certain time interval, or alternatively the construction of the fever curve 50 may be performed in parallel with the monitoring the subject 1 over said time interval. The processor arrangement 34 may construct the fever curve 50 based on the alternating pattern of shivering episodes 52 and sweating episodes 54 and the periodicity of this alternating pattern.

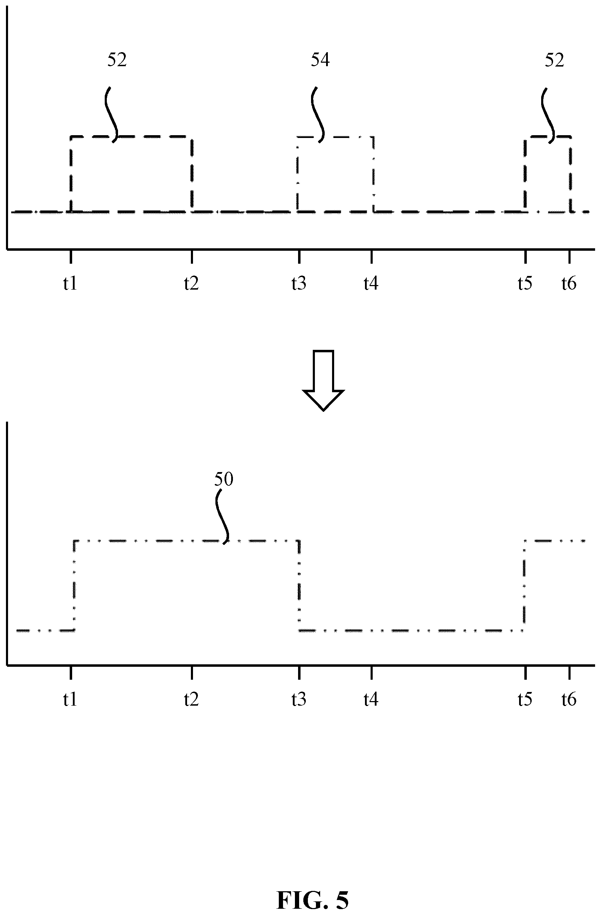

For example, as schematically depicted in FIG. 5, the processor arrangement 34 may receive an indication of the onset of a shivering episode 52 at T = t1, e.g. from the sensor arrangement 20 or through a manual input as previously explained, which shivering episode 52 terminates at T = t2. The upper graph shows intensity (a.u.) of the shivering episodes 52 and sweating episodes 54 (Y-axis) as a function of time (X-axis). The processor arrangement 34 further receives an indication of the onset of a sweating episode 54 at T = t3, e.g. from the sensor arrangement 20 or through a manual input as previously explained, which sweating episode 54 terminates at T = t4. The processor arrangement 34 further receives an indication of the onset of a further shivering episode 52 at T = t5, e.g. from the sensor arrangement 20 or through a manual input as previously explained, which further shivering episode 52 terminates at T = t4. The processor arrangement 34 may construct the fever curve 50 from the points in time at which a shivering episode 52 and a sweating episode 54 initiated, i.e. t1, t3 and t5 without factoring in the duration of each episode, which produces the resulting (surrogate) fever curve 50 as shown in FIG. 5, with CBT, or more accurately, projected change in CBT (Y-axis) as a function of time (X-axis). As can be seen from this curve, the onset of each shivering episode 52 is interpreted as an instantaneous rise in CBT, whereas the onset of each sweating episode in interpreted as an instantaneous drop in CBT. Of course, alternative approaches, e.g. the fitting of a sinusoidal curve based on the recorded points in time of the onset of respective shivering episodes 52 and sweating episodes 54 are also feasible. The processor arrangement 34 subsequently generates an output based on the thus constructed fever curve 50 in operation 117, e.g. for transmission to the user interface 41 or 42 and/or for storage in the data storage architecture 43, after which the method 100 terminates in operation 119.

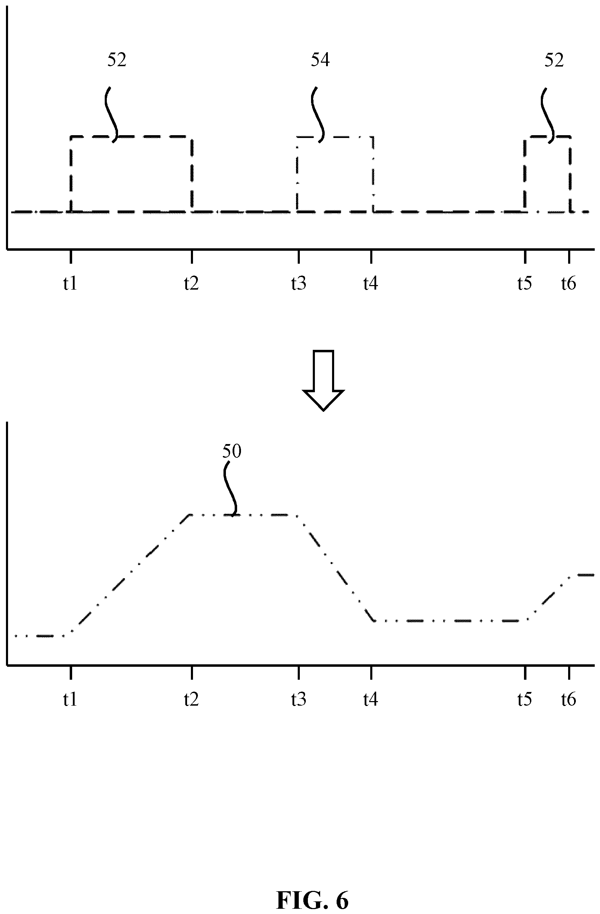

In a refinement, when constructing the fever curve 50 in operation 115, the processor arrangement 34 may factor in the duration of each of the shivering episodes 52 and sweating episodes 54. This is shown in FIG. 6, in which at the start of each shivering episode 52, the processor arrangement 34 assumes a gradual increase such as a linear increase of the CBT of the subject until the end of such a shivering episode 52, after which the CBT is assumed constant until the start of a subsequent sweating episode 54, at which point in time the processor arrangement 34 assumes a gradual decrease such as a linear increase of the CBT of the subject until the end of such a sweating episode 54 in its construction of the fever curve 50. Again, as an alternative, a sinusoidal curve may be fitted based on the start and end values of the respective start and end points (in time) of the respective shivering episodes 52 and sweating episodes 54.

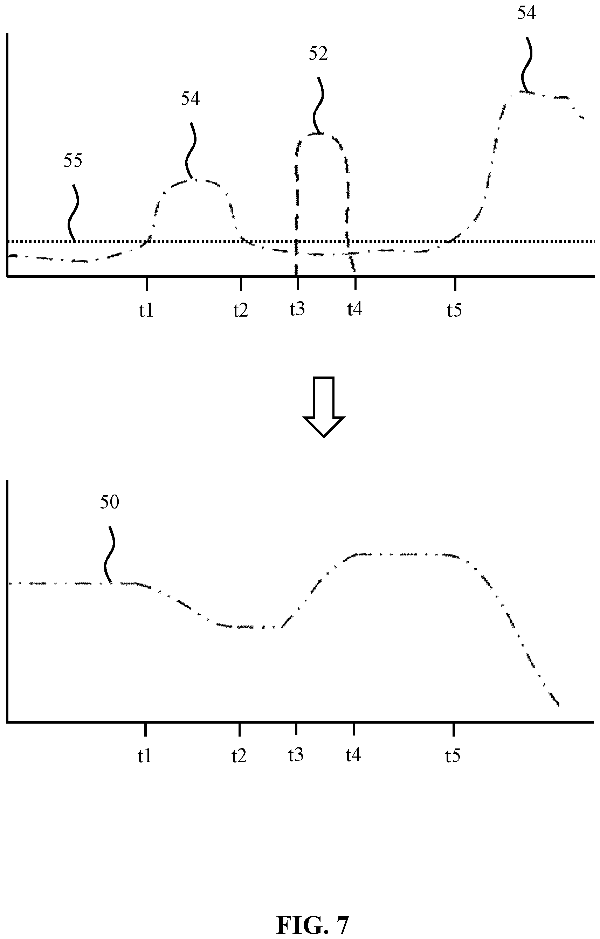

In a further refinement, as shown in FIG. 7, the processor arrangement 34 may take both the duration and the intensity of the shivering episodes 52 and sweating episodes 54 into consideration in the construction of the fever curve 50. In this approach, when the subject 1 is sweating heavily, i.e. has a high sweat rate, the reproduced approximation of the CBT of the subject 1 decreases faster than when the subject 1 sweats only a little, i.e. has a low sweat rate. As mentioned above, a sweating episode 52 may be detected upon the actual sweat rate exceeding a sweat base rate or threshold 55. Similarly, when the subject 1 is shivering heavily, the reproduced approximation of the CBT of the subject 1 increases faster than when the subject 1 only shivers a little. The intensity of a shivering episode may be determined using a power spectral density analysis as will be explained in more detail below. Since the intensity level of a shivering episode 52 or a sweating episode 54 can change over time, instead of the binary an/off approach in the construction of the fever curve 50 in FIG. 5, it thus becomes an integral as expressed in equation (1):

$$'temperature'(t) = T_0 + \int_0^t (c_1(intensity\ shivering) - c_2(intensity\ sweating))dt \qquad \text{(equation 1)}$$

In this equation, t is the time, T0 is the temperature at t=0, which may be measured, estimated, or may be a default value, and c1 and c2 are parameters that are patient- and environment- dependent (e.g. factoring in the degree thermal insulation of the subject 1, *vide infra*) and may even depend on the intensity of respectively shivering and sweating. If no independent CBT measurement is available, default values may be used for T0, c1 and c2. However, if such an independent CBT measurement becomes available, this measurement may be used to determine T0. Where multiple independent CBT measurements over a period of time become available, e.g. through continuous or spot check CBT measurements over a period of time during which shivering episodes 52 and sweating episodes 54 occur, c1 and c2 can also be estimated for the particular subject, environment, and shivering/sweating episode intensity levels during the CBT measurements. This may serve as a calibration of the fever curve 50 construction by the processor arrangement 34 once the CBT measurements have stopped. Hence, in this embodiment it is possible to provide a fever curve 50 including estimated absolute values for the subject's CBT on the Y-axis of the graph depicting the fever curve 50 as a function of time on the X-axis of this graph.

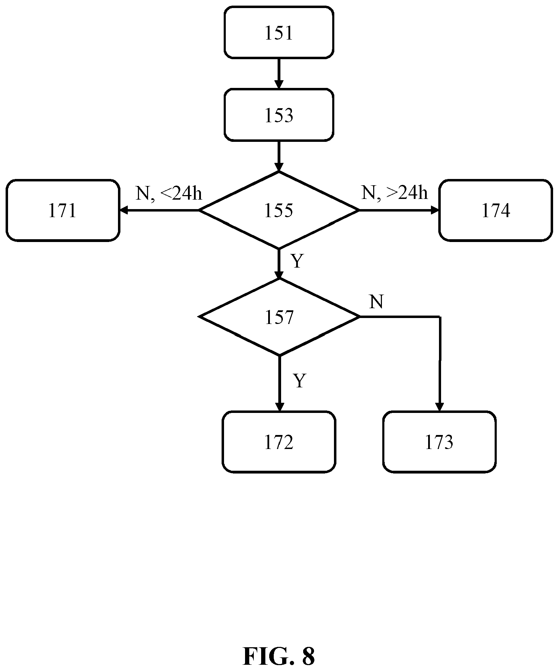

In a preferred embodiment, the processor arrangement 34 determines the type of fever from the recorded points in time at which the detected shivering episodes 52 and the sweating episodes 54 of the subject 1 occur, e.g. in addition to or instead of the generation of the fever curve 50, and includes this information in the output generated in operation 117, e.g. in addition to the fever curve 50 or as information derived from the fever curve 50 without including the actual fever curve 50, to provide a medical professional with additional insights in the underlying condition of the subject 1. This is explained in more detail with the aid of FIG. 8, which schematically depicts a decision tree that may be deployed by the processor arrangement 34 for determining the type of fever from the recorded points in time. The decision tree starts in operation 151, after which the processor arrangement 34 in operation 153 determines the periodicity of the fever curve 50 in operation 153, e.g. by determining the elapsed time between successive shivering episodes 52 separated by a sweating episode 54 or between successive sweating episodes 54 separated by a shivering episode 52 from the recorded points in time. If this periodicity is less than about 24 hours, as determined in operation 155, the processor arrangement 34 determines that the type of fever is a remittent fever, as symbolized by conclusion 171.

If on the other hand it is determined that this periodicity is about 24 hours, the processor arrangement 34 decides that the type of fever is a quotidian fever. The processor arrangement 34 may then further evaluate the elapsed time between a shivering episode 52 and a subsequent sweating episode 54 in operation 157 to determine the ability of the subject's body to fight the quotidian fever. Typically, when the subject's immune system is functioning properly, the duration of the elevated CBT or fever should be no longer than a few hours, during which the burst of parasites is attached by the subject's immune system. Hence, when the processor arrangement 34 concludes in operation 157 that the duration of a period of elevated CBT corresponds to an appropriate functioning immune system, the processor arrangement 34 may conclude that the fever is 'normal' quotidian fever as symbolized by conclusion 172, whereas if the processor arrangement 34 concludes in operation 157 that the duration of a period of elevated CBT exceeds a threshold value indicative of a compromised immune system, the processor arrangement 34 may conclude that the fever is quotidian fever in a weakened subject 1, as symbolized by conclusion 173.

Of course, the evaluation of the state of the subject's immune system is optional, in which case the determination of the elapsed time between a shivering episode 52 and a subsequent sweating episode 54 may be omitted from this evaluation. Consequently, recording of the points in time at which the sweating episodes 54 occur may be omitted and the evaluation of the fever curve 50 may be based on the elapsed time between successive shivering episodes 52 only, as long as these shivering episodes are separated by a sweating episode 54. Equally, recording of the points in time at which the shivering episodes 52 occur may be omitted and the evaluation of the fever curve 50 may be based on the elapsed time between successive sweating episodes 54 only, as long as these sweating episodes are separated by a shivering episode 52.

If the processor arrangement 34 determines in operation 155 that the periodicity of the fever in the subject 1 clearly exceeds 24 hours, the processor arrangement 34 may conclude that the fever is another type of fever as symbolized by conclusion 174, such as a tertian fever, a quartan fever, relapsing fever, undulant fever or the like. A further decision tree (not shown) may be implemented by the processor arrangement 34 to distinguish between such further types of fever.

In at least some embodiments in which the sensor arrangement 20 is used to collect subject monitoring data from which the fever curve 50 is constructed as explained above, additional sensors, e.g. additional sensors 223, may be used for a number of reasons. For example, the sweat sensor 222 of the wearable sensor 22 may further be configured to determine the concentration of an analyte of interest, e.g. cytokines or lactate, in the excreted sweat, which may be used by the processor arrangement 34 to further determine the cause of the fever, the severity of the disease, and/or efficacy of administered medication to combat the disease, which information can provide helpful insights for the medical caregiver in how to treat the subject 1, e.g. by administering different medication dosages, different medication and so on. Further additional sensors, which preferably but not necessarily are integrated in one or more wearable sensors 22, may provide physiological monitoring data such as heart rate, respiration rate, blood pressure, peripheral oxygen saturation and so on, which sensor data may also be included in the output generated by the processor arrangement 34 to further enhance the diagnostic potential of this output.

The sensor arrangement 20 may further comprise one or more additional sensors that provide auxiliary information to the processor arrangement 34 that allows the processor arrangement 34 to distinguish between fever-induced shivering episodes 52 and sweating episodes 54 or the occurrence of such episodes for different reasons, e.g. one or more motion sensors, heart rate monitors, blood pressure sensors or the like to detect increased subject activity, e.g. sports activity, strenuous activity such as walking up stairs and the like, which can cause sweating episodes that are triggered by the activity rather than a fever-induced lowering of the subject's temperature set point. An environmental sensor, e.g. an ambient temperature sensor or the like may be deployed to determine if the onset of a shivering or sweating episode is caused by environmental factors, e.g. a low or high ambient temperature, instead of because of illness. Using this auxiliary information, the processor arrangement 34 may determine whether a shivering episode 52 or a sweating episode 54 is a reliable indicator of an illness-induced body temperature change of the subject 1 such that the processor arrangement 34 only includes the point in time of such a shivering episode 52 or sweating episode 54 in the fever curve 50 if the processor arrangement 34 decides that the shivering episode 52 or the sweating episode 54 is a reliable indicator of an illness-induced body temperature change of said subject 1 rather than likely to be caused by other factors as signaled by the auxiliary information.

In yet another embodiment, the sensor arrangement 20 further comprises a CBT sensor, which may be an additional sensor 223 in the wearable sensor 22 or a separate (wearable) sensor. Including CBT information in the subject monitoring data allows for even more information to be obtained from the points in time at which shivering episodes 52 and/or sweating episodes 54 occur. Typically, CBT will go up due to shivering and down due to sweating, but there is a certain lag time between the occurrence of such a shivering or sweating episode and the accompanying change in CBT. This lag time can be used to get an insight in the subject's thermoregulatory ability. In addition, the CBT information may be used to calibrate the construction of the fever curve 50 by the processor arrangement 34, e.g. as a ground truth for training artificial intelligence or machine learning algorithms used by the processor arrangement 34 to construct the fever curve 50.

Another reason to add a CBT measurement is to distinguish between shivering due to an elevated set point (resulting in fever) and shivering due to the subject 1 being too cold (with a normal set point). This is in particular useful when establishing the start of a fever period. One easy way to distinguish between these two possibilities is to perform a single CBT measurement when the shivering has been detected for a suitable period of time (say 10 minutes). If the elevated set point is the cause, the temperature will already be elevated, whilst if the body is cold it will be depressed.

As is well-known per se, fever is the increase of body temperature during an infection. However, the mechanism underlying this fever is more complex and involves the host response to the infection. The immune system is activated by the source of infection and releases chemokines in the body resulting into an activation of cyclooxygenase (COX) resulting in a stimulation of 'heat gain thermo-effectors' (i.e. shivering) and an inhibition of 'heat loss thermo-effectors' (i.e. sweating). Normally only the core-body temperature is measured, and apart from the aforementioned impractical clinical integration, it only provides information of the increase of the subject's temperature set point. Therefore, in a preferred embodiment, the construction of the fever curve 50 further includes the determination of the shivering rate and the sweat rate of the subject 1 to provide an additional layer of diagnostically relevant information to the fever curve 50 as this for example provides information about the host's response, i.e. the immune response, to the source of the infection. In addition or as an alternative to the measurement of the CBT of the subject 1 to determine the lag between the shivering and sweating episodes and the induced changes in the subject's CBT as explained above, such a host response may be calculated from a number of parameters, such as the changes in the amount of time between successive shivering episodes 52, the changes in the amount of time between successive sweating episodes 54, the changes in the amount of time between a shivering episode 52 and a subsequent sweating episode 54 as well as the intensity and duration of such shivering episodes 52 and sweating episodes 54. The intensity of a sweating episode 54 may be based on a sweat rate determined with the sensor arrangement 20. The intensity of a shivering episode 52 may be determined based on a Fourier analysis and/or a spectral power analysis of the shivering data provided by the sensor arrangement 20. This may serve a number of purposes. Firstly, such an analysis of the shivering data provides a measurement of the trembling frequency of the subject 1, which allows the processor arrangement to make a distinction between unintended muscle movements, e.g. age-related tremors or the like, and shivering due to an elevated set point compared to the actual CBT of the subject 1. For example, hand tremors caused by Parkinson's disease can be as high as 9 Hz, which tremor amplitudes and acceleration amplitudes can reach 19 cm and 20 m/s$^2$ respectively. During fever or hyperthyreoses, the frequency increases up to 14 Hz, thereby allowing a differentiation between fever-related tremors and other types of tremors, such that the processor arrangement 34 can discard shivering episodes 52 caused by such other type of tremors and only consider fever-induced shivering episodes 52 in the construction of the fever curve 50, e.g. by using a bandpass filter or a high-pass filter with a suitable cutoff frequency, e.g. 10 Hz.

Secondly, the ratio of the signal strength of the shivering signal produced by the sensor arrangement 20 to the total sweat production or the sweat rate over time as monitored over a subject monitoring time interval may be used to construct a fever curve to enable prediction of fever severity and medical therapy response efficacy.

In a first step, fast Fourier analysis, e.g. amplitude or power spectrum signal analysis, of the shivering signal is performed to determine the strength of shivering. The power spectral density (PSD) of the shivering signal (PSD_Shivering) describes the power present in the signal as a function of frequency, per unit frequency. Power spectral density is commonly expressed in watts per Hertz (W/Hz). The total power present in the signal over a relevant frequency range (e.g. 10-20 Hz, or 5-15 Hertz) is then calculated.

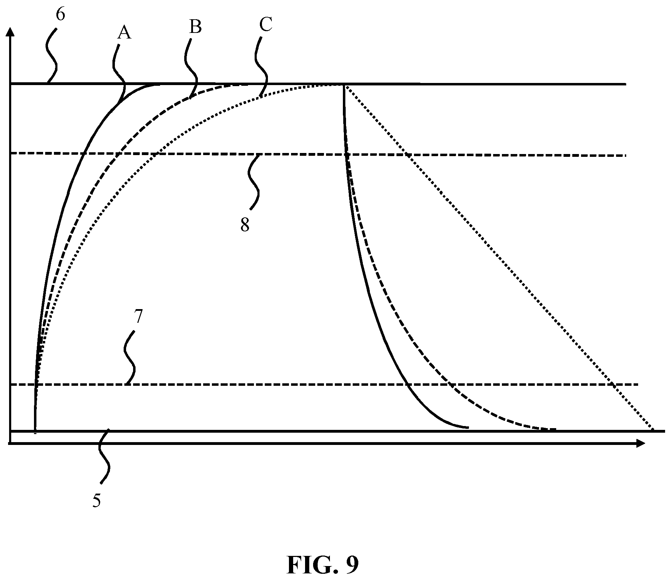

In a second step, the ratio of the shivering signal strength to the sweat rate (nL/s] is determined to calculate a fever index as per equations 2 and 3 below. Such a fever index may be an index at a given time (equation 2) or may be a time-integrated index (equation 3), i.e. the area under a fever index curve as shown in Fig. 9. If the fever index is integrated over time to calculate an accumulated fever index, (equation 2), this time-integrated index will provide clinical information pertaining to the fever episode or the fever interval intensity for a given time window of clinical interest. Another option is to calculate the first derivative of the fever index curve, which will then give information on the fever dynamics such as the speed of fever development or fever recovery.

$$Fever\ index\ \left[\frac{\frac{W}{Hz}}{\frac{nL}{s}}\right] = \frac{PSD\_Shivering}{Sweat\ rate} \qquad\qquad \text{(equation 2)}$$

$$Fever\ area\ index\ \left[\frac{\frac{W}{Hz}}{\frac{nL}{s}} \cdot s\right] = \int_{t=o}^{T} \frac{PSD\_Shivering}{Sweat\ rate}\ dt \qquad\qquad \text{(equation 3)}$$

The ratio is calculated because the onset and development of fever is normally proportional and directly linked to shivering, whereas fever can be assumed to be inversely proportional to sweat rate and drops when sweating. This is schematically depicted in FIG. 9, showing three fever index curves A-C progressing from a CBT having a normal or reference value 5 below an upper threshold 7 of such normal values to a strong fever value 6 above a critical threshold 8 as signaled by a shivering episode 52 and returning back to the normal or reference value 5 (as signaled by a sweating episode 54).

From the surrogate fever index curves A-C, time constants $\tau_{up}$ or $\tau_{down}$ can be determined or derived, in which, for example, $\tau_{up}$ is defined as the amount of time it takes for a fever curve 50 when starting from the reference value 5 to pass the critical threshold 8 and $\tau_{down}$ is defined as the amount of time it takes for a fever curve 50 when starting from the fever value 6 to pass the upper reference threshold 7. Alternatively, $\tau_{down}$ may be defined as the amount of time it takes for the fever to drop to 67% of its maximum value, similar to the calculation of decay times in electrical components such as capacitors. These time constants therefore carry clinical information about how fast fever develops and vanishes, e.g. after administration of medication. The fever curve as shown in FIG. 9 may be visualized on the user interface 41 or the user interface 42, for instance to enable clinical decision support for interventions or medication efficacy, e.g. to provide direct feedback to a medical caregiver on therapy response.

In an embodiment, the determination results of the shivering intensity and the sweat rate of the subject 1 may be scaled by the processor arrangement 34 based on available information regarding the thermal insulation of the subject 1. For example, the subject 1 during a shivering episode 52 may wear more clothes or add a blanket to keep warm, which can suppress the intensity of the shivering episode 52. Similarly, during a sweating episode 54, the subject 1 may shed clothing or blankets to cool down, thereby suppressing his or her sweat rate. To this end, the sensor arrangement 20 may provide information pertaining to a degree of thermal insulation of the subject to allow the processor arrangement 20 to determine at least one of a duration and a severity of each shivering episode 52 and sweating episode 54 as explained above as a function of the information pertaining to a degree of thermal insulation of the subject. Such information may be provided in any suitable manner. For example, determining the presence of a number blankets or clothing items, and/or the thickness of such blankets or clothing items could be derived from the image data provided by the camera 21. Alternatively, such information may be generated with the wearable sensor 22, for example by providing measurements of one or more of skin temperature, pressure, and ambient light, where in particular the pressure of a blanket pressing on the skin and ambient light, i.e. the light penetrating through the blanket and clothes, are independent of the core body temperature and are particularly useful parameters to monitor for this purpose.

The embodiments of the present invention can provide clinical insights into the thermoregulation capability of the body of the subject 1, e.g. to provide insights into the effect of administered medication, the severity of the disease, or assist in diagnosing the cause of fever. Such clinical insights can be useful in a number of care settings such as a hospital, hospital-to-home, or for home use, where it can assist in finding the right medication, dose, and timing, e.g. the clinical information provided by the processor arrangement 34 can aid the decision as to whether or not antibiotics are required to treat the medical condition of the subject 1, thereby reducing the risk that such antibiotics are administered unnecessarily, which is undesirable in the context of building up resistance to such antibiotics. However, it should be understood that use of the invention is not limited to medical care applications and may be utilized to get insight into the thermoregulation characteristics of the body of any subject, e.g. human or non-human, in general. In this context, the term subject as used in this application should not be interpreted as being limited to someone suffering from disease as the term is intended to apply to any subject of which the CBT is monitored in accordance with the teachings of the present application.

The above described embodiments of the method 100 executed by the processor arrangement 34 may be realized by computer readable program instructions embodied on a computer readable storage medium having, when executed on a processor arrangement 34 of a computing device 30, e.g. a patient monitoring terminal or the like, cause the processor arrangement 34 to implement any embodiment of the method 100. Any suitable computer readable storage medium may be used for this purpose, such as for example an optically readable medium such as a CD, DVD or Blu-Ray disc, a magnetically readable medium such as a hard disk, an electronic data storage device such as a memory stick or the like, and so on. The computer readable storage medium may be a medium that is accessible over a network such as the Internet, such that the computer readable program instructions may be accessed over the network. For example, the computer readable storage medium may be a network-attached storage device, a storage area network, cloud storage or the like. The computer readable storage medium may be an Internet-accessible service from which the computer readable program instructions may be obtained. In some embodiments, at least part of the computer readable program instructions may be incorporated in the processor arrangement 34 in the form of hardware.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. Measures recited in mutually different dependent claims can be advantageously combined.

**Claims**

1.  A computer-implemented method (100) for monitoring progression of a fever in a subject (1), the computer-implemented method comprising using a processor arrangement (34) for:

    receiving (103, 109) input data indicative of shivering episodes (52) and sweating episodes (54) as indicators of progression of a fever in said subject over a time interval;
    recording (105, 111) the respective points in time at which said shivering episodes and sweating episodes occur; and
    generating (115, 117) an output indicative of progression of fever in said subject based on an alternating pattern of said shivering episodes and sweating episodes from the recorded respective points in time of the respective shivering and/or sweating episodes.

2.  The computer-implemented method (100) of claim 1, further comprising using said processor arrangement for:

    determining (153) a periodicity of said alternating pattern of said detected shivering and sweating episodes (52, 54) based on their respective occurrence at different points in time during said time interval; and
    identifying (155) a type of fever from the determined periodicity of said alternating pattern.

3.  The computer implemented method (100) of claim 1 or 2, wherein receiving said input data (103, 109) comprises:

    receiving subject monitoring data from a sensor arrangement (20) for monitoring shivering and sweating of said subject as indicators of progression of a fever in said subject over said time interval; and
    processing said received subject monitoring data to detect shivering and sweating episodes (52, 54) and record the respective points in time at which said shivering episodes and sweating episodes occur.

4.  The computer-implemented method (100) of claim 3, further comprising using said processor arrangement for:

    determining at least one of a duration and a severity of each shivering episode and sweating episode; and
    calculating an indication of an intensity of the fever from the determined at least one of a duration and a severity of each shivering episode and sweating episode.

5.  The computer-implemented method (100) of claim 4, wherein said subject monitoring data comprises information pertaining to a degree of thermal insulation of the subject, and the computer-implemented method further comprises using said processor arrangement for:
    determining at least one of a duration and a severity of each shivering episode and sweating episode as a function of said information pertaining to a degree of thermal insulation of the subject.

6. The computer-implemented method (100) of any of claims 3-5, further comprising using said processor arrangement for:

determining a shivering frequency for each shivering episode (52); and
determining if said shivering episode is an indicator of progression of a fever in said subject based on the determined shivering frequency.

7. The computer-implemented method (100) of any of claims 3-6, further comprising using said processor arrangement for:
identifying a sweating episode (54) based on a sweat rate of said subject.

8. The computer-implemented method (100) of claim 7, further comprising using said processor arrangement for:

determining (107) a sweat base rate for said subject from the received subject monitoring data during a shivering episode (52); and
detecting a sweating episode (54) by detecting an actual sweat rate of said subject in the received subject monitoring data that exceeds the sweat base rate by a defined amount.

9. The computer-implemented method (100) of claim 7 or 8, further comprising using said processor arrangement for:

comparing an actual sweat rate of the subject against a defined sweat rate threshold; and
detecting a sweating episode upon the actual sweat rate exceeding the defined sweat rate threshold.

10. The computer-implemented method (100) of any of claims 7-9, further comprising using said processor arrangement for:

determining a signal strength of a signal pertaining to shivering episodes (52) over the time interval;
determining a total amount of sweat produced by said subject during the sweating episodes (54) over the time interval;
determining the subject's sweat rate over the time interval from the determined total amount of sweat produced by said subject;
calculating a ratio of said determined signal strength and determined sweat rate; and
including said calculated ratio in said output.

11. The computer-implemented method (100) of any of claims 1-10, wherein said subject monitoring data further comprises core body temperature data pertaining to a core body temperature of said subject measured over said time interval with a core body temperature sensor of said sensor arrangement, and the computer-implemented method optionally further comprising, with said processor arrangement, including the core body temperature data in said output.

12. The computer-implemented method (100) of any of claims 1-11, further comprising using said processor arrangement for:

receiving auxiliary information during said time interval through said communication interface arrangement, said auxiliary information comprising at least one of an ambient temperature to which the subject is exposed and a measurement of an activity level of the subject;
determining whether a shivering episode or a sweating episode is a reliable indicator of progression of a fever in said subject based on the actual auxiliary information at the point in time of said shivering episode or sweating episode; and
including a shivering episode or a sweating episode in generating said output only when said shivering episode or a sweating episode is determined to be a reliable indicator of progression of a fever in said subject.

13. A computer program product comprising a computer readable storage medium having computer readable program instructions embodied therewith for, when executed on a processor arrangement (34) of a subject monitoring system (10), cause the processor arrangement to implement the method (100) of any of claims 1-12.

14. A monitoring system (10) for monitoring a subject, the monitoring system comprising a processor arrangement (34) arranged to receive input data indicative of shivering episodes (52) and sweating episodes (54) of said subject as indicators of progression of a fever in said subject over a time interval, wherein the monitoring system further comprises the computer program product of claim 12 and the processor arrangement is arranged to execute said computer readable program instructions.

15. The monitoring system (10) of claim 14, further comprising a communication interface arrangement (32) arranged to receive said input data in the form of subject monitoring data from a sensor arrangement (20) for monitoring shivering and sweating of said subject as indicators of progression of a fever in said subject (1) and to forward said subject monitoring data to said processor arrangement (34), the monitoring system optionally comprising the sensor arrangement.

10

FIG. 1

**FIG. 2**

**FIG. 3**

100

FIG. 4

**FIG. 5**

**FIG. 6**

**FIG. 7**

**FIG. 8**

**FIG. 9**

Europäisches
Patentamt

European
Patent Office

Office européen
des brevets

**EUROPEAN SEARCH REPORT**

Application Number

EP 20 21 0971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/242850 A1 (ELLIS NATHAN [IE] ET AL) 30 August 2018 (2018-08-30) | 1-7,9, 11-15 | INV. A61B5/01 A61B5/11 A61B5/0537 |
| A | * paragraphs [0015] - [0033], [0049] - [0054], [0060] - [0064], [0068]; claims; figures * | 8,10 | |
| A | EP 2 526 856 A1 (KONINKL PHILIPS ELECTRONICS NV [NL]) 28 November 2012 (2012-11-28) * paragraphs [0036] - [0050]; claims; figures * | 1-15 | ADD. A61B5/00 |
| A | US 2019/117155 A1 (CROSS PEGGI S [US] ET AL) 25 April 2019 (2019-04-25) * paragraphs [0171] - [0176] * | 1-15 | |
| A | US 2018/249919 A1 (PONT DALTON [US] ET AL) 6 September 2018 (2018-09-06) * paragraphs [0019], [0077], [0086], [0097], [0108], [0119], [0127]; claims; figures * | 1-15 | |

TECHNICAL FIELDS
SEARCHED (IPC)

A61B

The present search report has been drawn up for all claims

| Place of search | Date of completion of the search | Examiner |
|---|---|---|
| The Hague | 15 May 2021 | Crisan, Carmen-Clara |

EPO FORM 1503 03.82 (P04C01)

## ANNEX TO THE EUROPEAN SEARCH REPORT
## ON EUROPEAN PATENT APPLICATION NO.

EP 20 21 0971

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2021

| Patent document cited in search report | | Publication date | Patent family member(s) | | Publication date |
|---|---|---|---|---|---|
| US 2018242850 | A1 | 30-08-2018 | US 2018242850 A1 | | 30-08-2018 |
| | | | WO 2018033799 A1 | | 22-02-2018 |
| EP 2526856 | A1 | 28-11-2012 | BR 112013029849 A2 | | 13-12-2016 |
| | | | CN 103561636 A | | 05-02-2014 |
| | | | EP 2526856 A1 | | 28-11-2012 |
| | | | EP 2713853 A1 | | 09-04-2014 |
| | | | JP 6249943 B2 | | 20-12-2017 |
| | | | JP 2014517758 A | | 24-07-2014 |
| | | | RU 2013157909 A | | 10-07-2015 |
| | | | US 2014088443 A1 | | 27-03-2014 |
| | | | WO 2012160500 A1 | | 29-11-2012 |
| US 2019117155 | A1 | 25-04-2019 | EP 3697293 A1 | | 26-08-2020 |
| | | | US 2019117155 A1 | | 25-04-2019 |
| | | | WO 2019079444 A1 | | 25-04-2019 |
| US 2018249919 | A1 | 06-09-2018 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• WO 2017172837 A1